# DEMANDE DE BREVET EUROPEEN

(11) **EP 1 466 588 A1**
(43) Date de publication de la demande: **13.10.2004**
(21) Numéro de dépôt: 04290590.1
(22) Date de dépôt: 04.03.2004
(51) Int. Cl.: A61K 7/48

(54) **Composition sous forme d'émulsion H/E contenant des cires, et son utilisation dans le domaine cosmétique**

(30) Priorité: 11.04.2003 FR 0304577
(71) Demandeur: L'OREAL, 75008 Paris (FR)
(72) Inventeur: Aubrun-Sonneville, Odile, 92160 Antony (FR); L'Alloret, Florence, 75013 Paris (FR)
(74) Mandataire: Rasson, Catherine

(57) **Abrégé**

L'invention se rapporte à une composition pour application topique se présentant sous forme d'une émulsion H/E contenant au moins une cire et au moins un polymère amphiphile particulier, et à l'utilisation de la dite composition, en particulier pour le soin, la protection et/ou le maquillage de la peau du corps ou du visage, des cils et/ou des lèvres, et/ou pour le soin des cheveux.

La composition obtenue peut être avantageusement exempte de tout tensioactif classiquement utilisé dans les émulsions H/E. Elle présente une très bonne stabilité et aussi de bonnes propriétés cosmétiques.

La composition se présente notamment sous forme de crème ou de lait.

La présente invention se rapporte aussi aux utilisations de ladite composition dans les domaines cosmétique et dermatologique, notamment pour le soin, la protection et/ou le maquillage de la peau et/ou des muqueuses.

## Description

L'invention se rapporte à une composition se présentant sous forme d'une émulsion H/E contenant au moins une cire et au moins un polymère amphiphile particulier, et à l'utilisation de la dite composition, en particulier pour le soin, la protection et/ou le maquillage de la peau du corps ou du visage, des cils et/ou des lèvres, et/ou pour le soin des cheveux.

Dans le domaine cosmétique, l'ajout de cires dans des compositions de soin de la peau permet d'obtenir des produits particulièrement adaptés au traitement des peaux sèches et des peau âgées. Les cires contribuent notamment à la nutrition de la peau et au lissage des rides et ridules. Elles peuvent être introduites aussi bien dans les émulsions E/H que dans les émulsions H/E. Généralement, dans le domaine cosmétique, les émulsions H/E sont préférées aux E/H en raison de leurs qualités cosmétiques jugées supérieures (légèreté, fraîcheur) et de leur plus grande flexibilité de formulation. Cependant, la présence de cires dans des formules H/E classiques entraîne une altération de leur stabilité, contrairement aux formules E/H. Par ailleurs, les propriétés cosmétiques des formules H/E contenant des cires sont encore jugées insuffisantes sur certains critères : consistance trop élevée, étalement difficile et présence d'un film gras.

Il subsiste donc le besoin de réaliser des émulsions H/E contenant des cires, stables et présentant des propriétés cosmétiques améliorées.

La demanderesse a découvert de façon inattendue que l'utilisation de polymères non réticulés particuliers, dérivés d'acide 2-acrylamido 2-méthylpropane sulfonique (AMPS), permettait de résoudre le problème à la base de l'invention et d'avoir une composition stable, ayant de bonnes propriétés cosmétiques et pouvant être avantageusement exempte de tensioactif émulsionnant classiquement utilisé dans les émulsions H/E, et donc bien tolérée par tout type de peaux.

Certes, il est connu par le document EP-A-1,069,142, des émulsions H/E contenant des polymères dérivés d'AMPS comportant une chaîne hydrophobe. Toutefois, les émulsions H/E décrites dans ce document ne contiennent pas de cires dont la présence est un facteur de déstabilisation de l'émulsion. En outre, l'utilisation de cires n'est décrite dans ce document que dans l'exemple 45 qui non seulement est une émulsion E/H et non H/E mais qui de plus contient un émulsionnant (Polyglyceryl-2 sesquistearate) qui peut se placer à l'interface des phases aqueuse et huileuse, le polymère non réticulé d'AMPS étant là comme épaississant.

Par ailleurs, les documents WO-A-02/43689, WO-A-02/44231, WO-A-02/44271, WO-A-02/44270, WO-A-02/43686, WO-A-02/44267 et WO-A-02/44230 décrivent des compositions cosmétiques, dermatologiques ou pharmaceutiques pour application topique, comprenant un copolymère amphiphile dérivé d'AMPS.
Toutefois, aucun de ces documents ne décrit des émulsions H/E contenant des cires.

La demanderesse a trouvé de manière surprenante que l'utilisation de polymères non réticulés particuliers décrits ci-après, caractérisés par des proportions déterminées en monomères permettaient d'atteindre le but de l'invention, c'est-à-dire d'obtenir des émulsions H/E contenant des cires, stables même en l'absence de tensioactifs classiques des émulsions H/E.

Par ailleurs, certains des polymères utilisés selon l'invention permettent d'obtenir des compositions ayant des textures particulièrement agréables et donc de très bonnes propriétés cosmétiques. Toutefois, ces polymères plus particuliers ne permettent pas seuls d'obtenir des émulsions stables, et la demanderesse a constaté de manière surprenante que l'association de ces polymères avec une ou plusieurs cires résolvait ce problème d'instabilité de l'émulsion.

Ainsi, non seulement les polymères sélectionnés permettent d'obtenir des émulsions contenant des cires, qui soient stables même en l'absence de tensioactifs classiques, mais en outre les cires permettent de stabiliser les émulsions contenant certains de ces polymères qui ont l'avantage de donner des émulsions de texture très cosmétique mais qui ont l'inconvénient de ne pas suffire pour obtenir seuls, une émulsion stable. L'association d'au moins une cire et d'au moins un polymère utilisé selon l'invention est donc particulièrement avantageuse pour obtenir des émulsions qui à la fois soient stables et présentent de bonnes propriétés cosmétiques.

L'invention a donc pour objet une composition pour application topique, constituée d'une émulsion H/E comprenant une phase huileuse dispersée dans une phase aqueuse, caractérisée en ce qu'elle contient au moins une cire et au moins un polymère amphiphile, non réticulé, comprenant de :
(a) 80 à 99 % en moles de motif acide 2-acrylamido 2-méthylpropane sulfonique (AMPS) de formule (I) suivante : 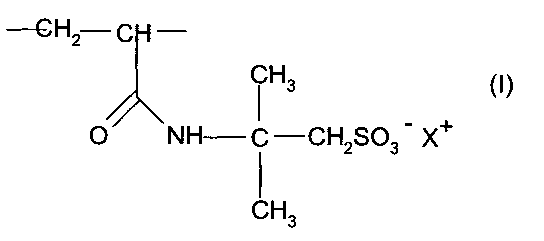 dans laquelle X⁺ est un proton, un cation de métal alcalin, un cation alcalino-terreux, l'ion ammonium ou un cation organique ; et
(b) 1 à 20 % en moles de motif de formule (II) suivante : 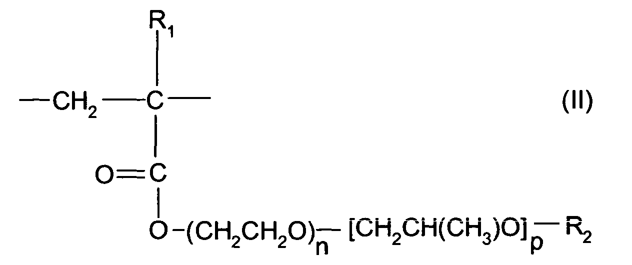
dans laquelle n et p indépendamment l'un de l'autre désignent un nombre entier allant de 0 à 24, de préférence de 1 à 24 et plus préférentiellement de 3 à 20, sous réserve que n + p soit inférieur à 25, de préférence inférieur à 20 et encore mieux inférieur à 15 ; R₁ désigne un atome d'hydrogène, un radical alkyle linéaire ou ramifié comportant de 1 à 6 atomes de carbone (C₁-C₆) (de préférence méthyle), et R₂ désigne un radical alkyle linéaire ou ramifié, comportant de 6 à 30 atomes de carbone (C₆-C₃₀), de préférence de 6 à 22 (C₆-C₂₂) et plus préférentiellement de 12 à 18 atomes de carbone (C₁₂-C₁₈).

La composition selon l'invention étant destinée à une application topique sur la peau ou les phanères, elle contient un milieu physiologiquement acceptable. On entend par "milieu physiologiquement acceptable", un milieu compatible avec la peau, les lèvres, les ongles, le cuir chevelu et/ou les cheveux.

La composition obtenue selon l'invention présente une bonne stabilité dans le temps, même à une température supérieure à la température ambiante (par exemple 45°C). Par « émulsions stables », on entend des émulsions qui, après 24 heures de stockage à toutes températures comprises entre 4°C et 50°C, ne présentent aucun changement macroscopique de couleur, d'odeur, de viscosité, ni de variation de pH.

La composition de l'invention se présente sous forme d'une crème plus ou moins fluide ou d'un lait, c'est-à-dire un produit souple par opposition à un produit solide tel qu'un stick. Ainsi, cette composition peut avoir une viscosité à la température ambiante (25°C) allant d'environ 1 à 250 poises (0,1 à 25 Pa.s) et de préférence d'environ 1 à 150 poises (1 à 15 Pa.s), cette viscosité étant mesurée avec un Rhéomat 180 à 25°C.

### Polymères non réticulés

Les polymères utilisés dans la composition de l'invention sont des copolymères non réticulés à base d'au moins un monomère hydrophile d'AMPS de formule (I) et d'au moins un monomère hydrophobe de formule (II). Ces polymères sont hydrosolubles ou hydrodispersibles, sous forme neutralisée ou partiellement neutralisée. Ils sont de préférence totalement neutralisés. Les polymères étant hydrosolubles, ils sont introduits dans la phase aqueuse de l'émulsion.

Par « polymères hydrosolubles ou hydrodispersibles », on entend des polymères qui, introduits dans de l'eau à une concentration égale à 1 % en poids, conduisent à une solution macroscopiquement homogène dont la transmittance de la lumière, à une longueur d'onde égale à 500 nm, à travers un échantillon de 1 cm d'épaisseur, est d'au moins 10%.

On entend par "polymère non réticulé", un polymère n'ayant pas réagi avec des agents de réticulation et donc ne comportant pas de groupes de réticulation. Il s'agit d'un polymère linéaire.

Les polymères de l'invention ont en général un masse moléculaire en poids allant de 10 000 à 10 000 000, plus préférentiellement de 100 000 à 8 000 000 et encore plus préférentiellement de 100 000 à 7 000 000.

Plus particulièrement, on utilise l'acide 2-acrylamido-2-méthylpropane-sulfonique (AMPS) de formule (I) sous ses formes partiellement ou totalement neutralisées.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères de l'invention peuvent notamment être choisis parmi les polymères amphiphiles non réticulés correspondant à la définition donnée ci-dessus, décrits de manière générale dans le document EP-A-1,069,142.

Comme indiqué ci-dessus, certains de ces polymères sont plus particulièrement intéressants pour obtenir une composition ayant des propriétés cosmétiques supérieures. Il s'agit plus particulièrement des polymères comprenant de :
(a) 85 à 99 % en moles, et de préférence de 90 à 99 % en moles, de motif acide 2-acrylamido 2-méthylpropane sulfonique (AMPS) de formule (I) telle qu'indiquée ci-dessus, et
(b) 1 à 15 % en moles et de préférence de 1 à 10 % en moles de motif de formule (III) suivante : 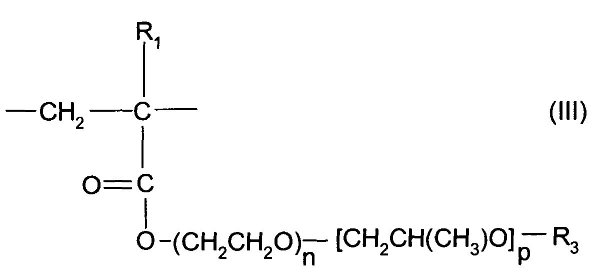
dans laquelle n et p indépendamment l'un de l'autre désignent un nombre entier allant de 7 à 24, de préférence de 8 à 24, sous réserve que n + p soit inférieur à 25, mieux inférieur à 20 et encore mieux inférieur à 15 ; R₁ désigne comme dans la formule (II) un atome d'hydrogène, un radical alkyle linéaire ou ramifié comportant de 1 à 6 atomes de carbone (C₁-C₆) (de préférence méthyle), et R₃ désigne un radical alkyle linéaire ou ramifié, comportant de 6 à 15 atomes de carbone (C₆-C₁₅), de préférence de 8 à 14 atomes de carbone (C₈-C₁₄).

Les polymères amphiphiles utilisés selon l'invention peuvent être obtenus selon les procédés classiques de polymérisation radicalaire en présence d'un ou plusieurs initiateurs tels que par exemple, l'azobisisobutyronitrile (AlBN), l'azobisdiméthylvaléronitrile, le chlorhydrate de 2,2-azobis-[2-amidinopropane] (ABAH=2,2-AzoBis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Les polymères peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction de polymérisation peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment obtenu des polymères utilisés selon l'invention à partir d'acide 2-acrylamido-2-méthylpropane-sulfonique (AMPS) ou l'un de ses sels de sodium ou d'ammonium, avec un ester de l'acide méthacrylique ou acrylique, de préférence un ester de l'acide méthacrylique, et
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL® T-200 de la société HOECHST/CLARIANT).

Comme polymères amphiphiles préférés utilisés selon la présente invention, on peut citer le copolymère obtenu à partir de 91,5 % en moles d'AMPS et 8,5 % en moles de méthacrylate d'alcool en C₁₂-C₁₄ comportant 7 groupes oxyéthylénés (Genapol LA-070) (polymère I) ; le copolymère obtenu à partir de 92,65 % en moles d'AMPS et de 7,35 % en moles de méthacrylate d'alcool en C₁₆-C₁₈ comportant 8 groupes oxyéthylénés (Genapol T-080) (polymère II) ; et le mélange de ces polymères. Ces polymères sont tous appropriés pour donner une composition stable en présence de cire. Ces polymères permettent en outre d'obtenir une composition ayant des propriétés cosmétiques particulièrement agréables. Ainsi, les crèmes obtenues sont très agréables à l'application et elles laissent un film qui est ni gras ni collant sur la peau. De plus, elles laissent la surface cutanée veloutée, et ont de bonnes propriétés d'hydratation.

La quantité de polymère(s) amphiphile(s) dans la composition de l'invention dépend du polymère utilisé. Elle peut aller par exemple, en matière active, de 0,05 à 20 % en poids, de préférence de 0,1 à 15 % en poids, mieux de 0,2 à 10 % en poids et encore mieux de 0,25 à 5 % en poids par rapport au poids total de la composition.

Selon un mode particulier de réalisation de l'invention, la composition selon l'invention peut être substantiellement exempte de tensioactif émulsionnant habituellement utilisé dans les émulsions H/E. On entend par " substantiellement exempte de tensioactif' une composition contenant moins de 0,5 % en poids et de préférence moins de 0,3 % en poids d'agent tensioactif émulsionnant par rapport au poids total de la composition, ou même étant totalement exempte de tout agent émulsionnant.

### Phase huileuse

La composition de l'invention contient au moins une cire généralement présente dans la phase huileuse. La quantité de cire(s) dans la composition de l'invention peut aller par exemple de 0,05 à 10 % en poids, mieux de 0,1 à 15 % en poids et encore mieux de 0,5 à 10 % en poids par rapport au poids total de la composition.

Par « cires », on entend des corps gras solide à température ambiante (25°C) et ayant une température de fusion supérieure à 30°C (mesurée par D.S.C) et mieux supérieure à 40°C.

Les cires, au sens de la demande, sont celles généralement utilisées dans les domaines cosmétique et dermatologique ; elles peuvent être hydrocarbonées, siliconées et/ou fluorées, comportant éventuellement des fonctions ester ou hydroxyle. On peut utiliser par exemple des cires minérales ; des cires d'origine animale ; des cires d'origine végétale ; les huiles hydrogénées concrètes à 25°C, les esters gras et les glycérides concrets à 25°C, les cires synthétiques, les cires de silicone et leurs mélanges.

Comme cires utilisables dans la composition de l'invention, on peut citer par exemple les cires microcristallines ; les cires de paraffine ; les cires de lignite ; la cérésine ; l'ozokérite ; la cire de montan ; la cire d'abeille ; la lanoline et ses dérivés ; les cires de Candellila, d'Ouricurry, de Carnauba, du Japon ; le beurre de cacao ; l'huile de palme sous forme de pâte à 20°C ; les cires de fibres de lièges ou de canne à sucre ; les huiles hydrogénées concrètes à 25°C ; les esters gras et les glycérides concrets à 25°C ; les cires de polyéthylène et les cires obtenues par synthèse de Fischer-Tropsch ; les cires de silicone, et leurs mélanges.

Selon un mode préféré de réalisation de l'invention, on utilise au moins une cire choisie parmi parmi la cire de Camauba, la cire d'abeille, l'huile de palme sous forme de pâte à 20°C, les cires de polyéthylène ayant une température de fusion commençante supérieure à 65°C, les cires microcristallines ayant une température de fusion commençante supérieure à 65°C, et leurs mélanges.

Outre les cires, la phase huileuse de la composition selon l'invention peut contenir au moins un corps gras choisi parmi les huiles liquides à température ambiante (20-25°C), volatiles ou non, les gommes et les corps gras pâteux, d'origine animale, végétale, minérale ou synthétique, et leurs mélanges. Ces corps gras sont physiologiquement acceptables.

Selon un mode préféré de réalisation de l'invention, la phase huileuse contient au moins une huile. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité et les fractions liquides de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- des alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles soit linéaires soit cycliques comme les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation de l'invention, la phase huileuse de la composition contient au moins une huile choisie parmi les hydrocarbures linéaires ou ramifiés, d'origine minérale ou synthétique, tels que l'huile de Parléam, parmi les huiles de silicone volatiles, notamment les cyclopolydiméthylsiloxanes, parmi les huiles végétales telles que les fractions liquides de beurre de karité, et parmi les esters tels que le palmitate d'octyle.

Les autres corps gras pouvant être présents dans la phase huileuse sont par exemple les acides gras comportant de 8 à 30 atomes de carbone, comme l'acide stéarique, l'acide laurique, l'acide palmitique ; les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations "KSG" par la société Shin-Etsu, sous la dénomination "Trefil" par la société Dow Corning ou sous les dénominations "Gransil" par la société Grant Industries ; les pâtes telles que Petrolatum ; et leurs mélanges.

Selon un mode particulier de réalisation de l'invention, la composition contient au moins une gomme de silicone dont la présence améliore encore les qualités cosmétiques de la composition. On utilise de préférence une ou plusieurs gommes de silicone ayant un poids moléculaire inférieur à 1 500 000, telle qu'un polydiméthylsiloxane (nom INCI : dimethicone), un polyphénylsiloxane, ou un polyhydroxysiloxane (nom INCI : dimethiconol). Quand elle est présente, la quantité de gomme de silicone peut aller par exemple de 0,01 à 10 % en poids, de préférence de 0,1 à 3 % en poids par rapport au poids de la composition finale.

La phase huileuse comprenant tous les corps gras et les adjuvants lipophiles éventuellement présents est présente dans la composition selon l'invention en une quantité allant généralement de 10 à 75 % et de préférence de 15 à 70 % en poids par rapport au poids total de la composition.

### Phase aqueuse

La phase aqueuse de la composition de l'invention peut aller de 90 à 25 % en poids et de préférence de 85 à 30 % en poids par rapport au poids total de la composition. Elle contient au moins de l'eau. Elle peut contenir, outre l'eau, un ou plusieurs composés miscibles à l'eau ou au moins en partie miscibles à l'eau, comme les polyols ; les mono-alcools inférieurs en C₂ à C₈, tels que l'éthanol et l'isopropanol ; et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylène glycol, l'hexylène glycol, le pentylène glycol et les polyéthylène glycols comme le PEG-8, le sorbitol, les sucres comme le glucose.

Le ou les solvants peuvent être présents en une quantité allant de 0,1 à 30 % en poids par rapport au poids total de la composition.

### Adjuvants

De façon connue, la composition de l'invention peut contenir également des adjuvants habituels dans les domaines cosmétique et/ou dermatologique, tels que les actifs, les conservateurs, les antioxydants, les agents complexants, les ajusteurs de pH (acides ou basiques), les parfums, les charges, les bactéricides, les absorbeurs d'odeur, les matières colorantes (pigments et colorants), les tensioactifs, les polymères et encore les vésicules lipidiques. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les vésicules lipidiques.

Comme polymères utilisables dans la composition selon l'invention, autres que les polymères utilisés selon l'invention, on peut citer notamment les polymères hydrophiles tels que les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Goodrich ; les polyacrylamides ; les polymères dérivés de l'acide 2-acrylamido-2-méthylpropane sulfonique tels que le produit commercialisé par Clariant sous la dénomination Hostacerin AMPS (nom INCI : ammonium polyacryldimethyltaurate) ; les copolymères anioniques réticulés d'acrylamide et d'AMPS, se présentant sous la forme d'une émulsion, tels ceux commercialisés sous le nom de SEPIGEL 305 (nom C.T.F.A. : Polyacrylamide C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les copolymères acrylate/acrylonitrile tels que le HYPAN SS201 commercialisé par la société Kingston ; les polymères neutres synthétiques tels que la poly-N-vinylpyrrolidone ; les polysaccharides comme les gommes de guar, de xanthane et les dérivés cellulosiques ; les dérivés siliconés hydrosolubles ou hydrodispersibles comme les silicones acryliques, les silicones polyéthers et les silicones cationiques ; les composés inorganiques tels que les smectites, les hectorites modifiées ou non telles que les produits BENTONE commercialisés par la société Rheox, les produits LAPONITE commercialisés par la société Southern Clay Products, le produit VEEGUM HS commercialisé par la société R.T.Vanderbilt ; et leurs mélanges. La quantité de ces polymères peut aller par exemple de 0,05 à 3 % en poids par rapport au poids total de la composition.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon@ telles que Nylon-1 ou Polyamide 12, vendues sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Comme actifs, on peut utiliser notamment les vitamines (A, B3, C, E, K, PP...) seules ou en mélange ainsi que leurs dérivés ; les agents kératolytiques et/ou desquamants tels que l'acide salicylique et ses dérivés, les alpha-hydroxyacides comme l'acide lactique, l'acide citrique et l'acide glycolique, l'acide ascorbique et ses dérivés , les agents anti-inflammatoires ; les agents apaisants tels que l'allantoïne ; les agents dépigmentants ; les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les agents matifiants ; les agents anti-chute et/ou repousse des cheveux ou encore les agents anti-rides et leurs mélanges.

Les filtres solaires peuvent être choisis parmi les filtres UV organiques, tels que les composés suivants :
- Les dérivés salicyliques et notamment l'éthylhexyl salicylate (ou salicylate d'éthyl hexyle) vendu sous le nom commercial NEO HELIOPAN OS par HAARMANN et REIMER ;
- Les dérivés du dibenzoylméthane et notamment le Butyl méthoxydibenzoylmethane vendu notamment sous le nom commercial PARSOL 1789 par HOFFMANN LA ROCHE ;
- Les dérivés cinnamiques et notamment l'éthylhexyl méthoxycinnamate vendu notamment sous le nom commercial PARSOL MCX par HOFFMANN LA ROCHE;
- Les dérivés de β,β'-diphénylacrylate et notamment l'octocrylène (α-cyano-β,β-diphénylacrylate de 2-éthylhexyle) vendu notamment sous le nom commercial UVINUL N539 par BASF ;
- L'acide phenylbenzimidazole sulfonique ;
- Les dérivés du benzylidène camphre, et notamment l'acide téréphthalylidène dicamphosulfonique (Terephthalylidene Dicamphor Sulfonic) fabriqué sous le nom MEXORYL SX par CHIMEX, et le 4-méthylbenzylidène camphre vendu sous le nom commercial EUSOLEX 6300 par MERCK ;
- Les dérivés de la benzophénone, et notamment la Benzophenone-3 ou Oxybenzone, vendue sous le nom commercial UVINUL M40 par BASF ; la Benzophenone-4 vendue sous le nom commercial UVINUL MS40 par BASF ; la Benzophenone-5 ;
- Les dérivés du phényl benzimidazole, et notamment le Benzimidazilate vendu sous le nom commercial commercial NEO HELIOPAN AP par HAARMANN et REIMER ;
- Les dérivés de la triazine, et notamment l'anisotriazine vendu sous le nom commercial TINOSORB S par CIBA GEIGY ; l'éthylhexyl triazone vendu notamment sous le nom commercial UVINUL T150 par BASF ; et le diéthylhexyl Butamido Triazone vendu sous le nom commercial UVASORB HEB par SIGMA 3V ;
- Le méthylène bis-benzotriazolyl tétraméthylbutylphenol ;
- Les dérivés du phényl benzotriazole, et notamment le Drometrizole Trisiloxane vendu sous le nom commercial SILATRIZOLE par RHODIA CHIMIE ; ou le Methylène bis-Benzotriazolyl Tetramethylbutylphénol, vendu sous forme sodide sous le nom commercial "MIXXIM BB/100" par FAIRMOUNT CHEMICAL ou sous forme micronisé en dispersion aqueuse sous le nom commercial "TINOSORB M" par CIBA SPECIALTY CHEMICALS ;
- et leurs mélanges.

Les filtres solaires peuvent être choisis aussi parmi les filtres physiques. Comme filtres physiques pouvant être ajoutés dans la composition de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

La quantité d'actifs dépend du but recherché. Le ou les actifs peuvent être par exemple présents en une concentration allant de 0,001 à 20 %, de préférence de 0,01 à 10 % en poids et mieux de 0,05 à 5 % du poids total de la composition.

Bien entendu, l'homme de l'art veillera à choisir le ou les éventuels composés à ajouter à la composition selon l'invention et leurs quantités, de manière telle que les propriétés avantageuses attachées intrinsèquement à la composition conforme à l'invention ne soient pas ou substantiellement pas, altérées par l'addition envisagée.

La composition selon l'invention peut se présenter notamment sous forme d'une crème plus ou moins fluide ou d'un lait, et elle peut constituer notamment une composition cosmétique ou dermatologique. Elle trouve alors son application dans un grand nombre de traitements notamment cosmétiques de la peau, y compris du cuir chevelu, des cheveux, des ongles, et/ou des muqueuses, en particulier pour le soin, la protection et/ou le maquillage de la peau du corps ou du visage, des cils et/ou des lèvres, et/ou pour le soin des cheveux (par exemple masque pour cheveux).

Aussi, la présente invention a pour objet l'utilisation cosmétique d'une composition cosmétique telle que définie ci-dessus, pour le soin, la protection et/ou le maquillage de la peau et/ou des lèvres et/ou pour le soin des cheveux.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition cosmétique telle que définie ci-dessus.

Par ailleurs, la composition selon l'invention est bien tolérée par les peaux sensibles. Ainsi, la présente invention a encore pour objet l'utilisation cosmétique d'une composition cosmétique telle que définie ci-dessus, pour le soin, la protection et/ou pour le maquillage des peaux sensibles.

Les compositions selon l'invention sont préparées de manière classique : le polymère est solubilisé dans la phase aqueuse. La phase huileuse est chauffée jusqu'à une température où tous les constituants huileux solides deviennent liquides. A cette température, la phase huileuse est introduite sous agitation dans la phase aqueuse, neutralisée ou non neutralisée. L'émulsion peut être réalisée avec des moyens d'homogénéisation classiques, comme par exemple une turbine rotor-stator.

L'exemple ci-après de compositions selon l'invention est donné à titre d'illustration et sans caractère limitatif. Les quantités y sont données en % en poids, sauf mention contraire.

Exemple 1 selon l'invention : Emulsion comprenant une cire et un copolymère amphiphile d'AMPS portant 8,5 % (en mole) de chaînes latérales C₁₂₋₁₄(OE)₇

### Phase huileuse

- Cire d'abeille 2 %
- Huile de Parleam 10,8 %
- Pentacyclomethicone 16 %
- Palmitate d'octyle 3 %
- Fraction liquide de beurre de karité 2 %
- Acétate de tocophérol 0,2 %
- Alcool cétylique 3 %
- Alcool stéarylique 3 %

### Phase Aqueuse

- Conservateur 1 %
- Triéthanolamine à 10% dans l'eau 0,05 %
- Copolymère d'AMPS et de méthacrylate de Genapol LA-070 (8,5 % en mole) 0,7 %
- Eau qsp 100 %

Mode opératoire : Le copolymère amphiphile d'AMPS est solubilisé pendant 2 heures sous agitation dans la phase aqueuse à 25°C ; la solution obtenue est macroscopiquement homogène. L'émulsion est préparée par introduction lente de la phase huileuse préalablement chauffée à 70°C, dans la phase aqueuse sous agitation à l'aide d'un homogénéisateur de type Moritz sous une vitesse d'agitation de 2000 RPM pendant 15 minutes.

La composition obtenue se présente sous la forme d'une crème souple de viscosité 39 Poises (soit 3,9 Pa.s) (mesure au Rhéomat180 avec un mobile 4 et à un gradient de cisaillement de 200 s⁻¹ après 10 minutes à 25°C), de pH 5,6. L'émulsion est fine au microscope. Au bout de deux mois à température ambiante et à 45°C, l'émulsion reste fine et conserve le même niveau de viscosité. Elle ne présente pas de signes de déstabilisation macroscopique.

### Tests cosmétiques :

L'exemple 1 a fait l'objet d'un test sensoriel sur 7 femmes, de type peau sèche ou mixte-sèche, âgées de plus de 35 ans. La crème a été appliquée par une esthéticienne sur le visage des femmes par demi-visage. Le produit est jugé comme ayant un effet adoucissant et assouplissant incontestable. Sa texture est ferme et fond rapidement lors de l'étalement. L'application est facile, la crème glisse bien et pénètre lentement sans effet frein ni collant. Au toucher, il reste sur la peau un léger film doux légèrement cireux, qui donne une impression de protection.

Exemple 2 selon l'invention : Emulsion comprenant une cire et un copolymère amphiphile d'AMPS portant 8,5 % (en mole) de chaînes latérales C₁₂₋₁₄(OE)₇

### Phase huileuse

- Vaseline (point de fusion 53-58°C) 3 %
- Gomme de silicone 7 %
- Huile de Parleam 15,8 %
- Pentacyclomethicone 9 %
- Palmitate d'octyle 3 %
- Huile de palme (triglycérides d'acides palmitique-oléique-stéarique 40/10/50) (point de fusion 37°C)(cire) 2 %
- Alcool cétylique 1 %
- Alcool stéarylique 13 %

### Phase Aqueuse

- Conservateur 0,6 %
- Triéthanolamine à 10% dans l'eau 0,06 %
- Glycérine 5 %
- Copolymère d'AMPS et de méthacrylate de Genapol LA-070 (8,5 % en mole) 0,8 %
- Eau qsp 100 %

Mode opératoire : identique à celui de l'exemple 1.

La composition se présente sous la forme d'une crème souple de viscosité 27 Poises (2,7 Pa.s) (mesure au Rhéomat 180 avec un mobile 3 et à un gradient de cisaillement de 200 s⁻¹ après 10 minutes à 25°C), de pH 6.5. L'émulsion est fine au microscope. Au bout de deux mois à température ambiante et à 45°C, l'émulsion reste fine, et conserve le même niveau de viscosité. Elle ne présente pas de signes de déstabilisation macroscopique.

Exemple 3 selon l'invention: Emulsion comprenant une cire et un copolymère amphiphile d'AMPS portant 7,35% (en mole) de chaînes latérales C₁₆₋₁₈(OE)₈

### Phase huileuse

- Huile de Parleam 15,8 %
- Pentacyclomethicone 16 %
- Palmitate d'octyle 3 %
- Fraction liquide de beurre de karité 2 %
- Cire d'abeille 1 %
- Acétate de tocophérol 0,2 %
- Alcool cétylique 1 %
- Alcool stéarylique 1 %

### Phase Aqueuse

- Conservateur 1 %
- Triéthanolamine à 10% dans l'eau 0,03 %
- Copolymère d'AMPS et de méthacrylate de Genapol LA-080 (7,35 % en mole) 0,5 %
- Eau qsp 100 %

Mode opératoire : identique à celui de l'exemple 1

La composition se présente sous la forme d'une crème souple de viscosité 13,4 Poises (1,34 Pa.s) (mesure au Rhéomat 180 avec un mobile 3 et à un gradient de cisaillement de 200 s⁻¹ après 10 minutes à 25°C), de pH 5.8. L'émulsion est assez fine au microscope. Au bout de deux mois à température ambiante et à 45°C, l'émulsion reste assez fine, et conserve le même niveau de viscosité. Elle ne présente pas de signes de déstabilisation macroscopique.

Exemple 4 selon l'invention : Emulsion comprenant une cire et un copolymère amphiphile d'AMPS portant 7,35 % (en mole) de chaînes latérales C₁₆₋₁₈(OE)₈

### Phase huileuse

- Huile de Parleam 15,8 %
- Pentacyclomethicone 16 %
- Palmitate d'octyle 3 %
- Fraction liquide de beurre de karité 2 %
- Cire d'abeille 1 %
- Acétate de tocophérol 0,2 %
- Alcool cétylique 1 %
- Alcool stéarylique 1 %

### Phase Aqueuse

- Conservateur 0,6 %
- Triéthanolamine à 10% dans l'eau 0,03 %
- Glycérine 5 %
- Copolymère d' AMPS et de méthacrylate de Genapol T-080 (7,35 % en mole) 0,5 %
- Eau qsp 100 %
Poudre de Nylon 3 %

Mode opératoire : identique à celui de l'exemple 1, avec ajout de la poudre de nylon après préparation de l'émulsion.

La composition se présente sous la forme d'une crème souple de viscosité 18,5 Poises (1,85 Pa.s) (mesure au Rhéomat 180 avec un mobile 3 et à un gradient de cisaillement de 200 s⁻¹ après 10 minutes à 25°C), de pH 6. L'émulsion est assez fine au microscope. Au bout de deux mois à température ambiante et à 45°C, l'émulsion reste assez fine, et conserve le même niveau de viscosité. Elle ne présente pas de signes de déstabilisation macroscopique.

Exemple 5 selon l'invention : Emulsion comprenant une cire et un copolymère amphiphile d'AMPS portant 8,5% (en mole) de chaînes latérales C₁₂₋₁₄(OE)₇

### Phase huileuse

- Cire d'abeille 2 %
- Huile de Parleam 13 %

### Phase Aqueuse

- Conservateur 1 %
- Triéthanolamine à 10% dans l'eau 0,06 %
- Copolymère d'AMPS et de méthacrylate de Genapol LA-070 (8,5% en mole) 1 %
- Eau qsp 100 %

Mode opératoire: identique à celui de l'exemple 1.

La composition obtenue présente sous la forme d'un lait (pH~6,1). La taille moyenne des gouttes est de 10 µm. Sa viscosité mesurée au Rhéomat 180 à 25°C à un gradient de cisaillement de 200 s⁻¹ au mobile 2 est de 0,13 Pa.s. Après 24 heures à 25°C, cette émulsion reste stable.

Exemple comparatif 1 : Emulsion sans cire contenant un copolymère amphiphile d'AMPS portant 8,5% (en mole) de chaînes latérales C₁₂₋₁₄(OE)₇

### Phase huileuse

- Cyclopentadiméthylsiloxane 6 %
- Huile de Parleam 9 %

### Phase Aqueuse

- Conservateur 1 %
- Triéthanolamine à 10% dans l'eau 0,06 %
- Copolymère d'AMPS et de méthacrylate de Genapol LA-070 (8,5% en mole) 1 %
- Eau qsp 100 %

Mode opératoire : identique à celui de l'exemple 1.

La formule se présente sous la forme d'un lait qui se déstabilise instantanément avec l'apparition d'un phénomène de crèmage (séparation d'une partie de la phase huileuse). Cette émulsion comprenant un copolymère amphiphile d'AMPS portant 8,5% (en mole) de chaînes latérales C_{12 14}(OE)₇ est instable en l'absence de cire, alors que les exemples 1, 2 et 5 selon l'invention qui comportent le même polymère et une cire est stable.

Exemple comparatif 2 : Emulsion avec cire contenant un copolymère amphiphile d'AMPS portant 3,55 % (en mole) de chaînes latérales C₁₆₋₁₈(OE)₂₅

### Phase huileuse

- Huile de Parleam 15,8 %
- Pentacyclomethicone 16 %
- Palmitate d'octyle 3 %
- Fraction liquide de beurre de karité 2 %
- Cire d'abeille 1 %
- Acétate de tocophérol 0,2 %
- Alcool cétylique 1 %
- Alcool stéarylique 1 %

### Phase Aqueuse

- Conservateur 1 %
- Triéthanolamine à 10% dans l'eau 0,03 %
- Copolymère d'AMPS et de méthacrylate de Genapol T-250 (3,55 % en mole) 0,5 %
- Eau qsp 100 %

L'émulsion obtenue est hétérogène et elle présente une phase huileuse qui se sépare de la phase aqueuse et surnage au-dessus de l'émulsion, alors que l'exemple 3 en tous points analogues à cet exemple à l'exception du polymère utilisé est parfaitement stable. Cet exemple montre que les polymères comportant 25 groupes oxyéthylénés ne permettent pas d'obtenir une émulsion H/E stable.

Exemple comparatif 3 : Emulsion avec cire contenant un copolymère amphiphile d'AMPS portant 58,2 % (en mole) de chaînes latérales C₁₂₋₁₄(OE)₇

### Phase huileuse

- Huile de Parleam 15,8 %
- Pentacyclomethicone 16 %
- Palmitate d'octyle 3 %
- Fraction liquide de beurre de karité 2 %
- Cire d'abeille 1 %
- Acétate de tocophérol 0,2 %
- Alcool cétylique 1 %
- Alcool stéarylique 1 %

### Phase Aqueuse

- Conservateur 1 %
- Triéthanolamine à 10% dans l'eau 0,03 %
- Copolymère d'AMPS et de méthacrylate de Genapol LA-070 (58,2 % en mole) 0,5 %
- Eau qsp 100 %

L'émulsion obtenue est hétérogène et présente un phénomène de crémage au bout de 24 heures à température ambiante (séparation des phases aqueuse et huileuse), alors que les exemples 1, 2 et 5 qui comportent un polymère analogue mais avec 8,5 % de chaîne hydrophobe sont parfaitement stables. Cet exemple montre que les polymères comportant plus de 25 % de chaînes hydrophobes ne permettent pas d'obtenir une émulsion H/E stable.

## Revendications

1. Composition pour application topique, constituée d'une émulsion H/E comprenant une phase huileuse dispersée dans une phase aqueuse, **caractérisée en ce qu'**elle contient au moins une cire et au moins un polymère amphiphile, non réticulé, comprenant de :
(a) 80 à 99 % en moles de motif acide 2-acrylamido 2-méthylpropane sulfonique (AMPS) de formule (I) suivante : 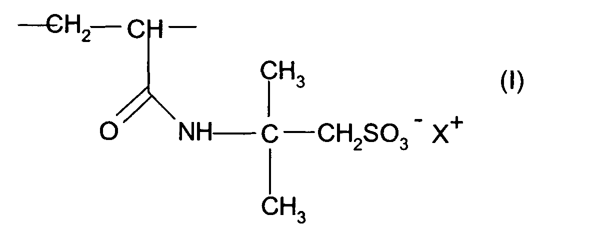 dans laquelle X⁺ est un proton, un cation de métal alcalin, un cation alcalino-terreux, l'ion ammonium ou un cation organique ; et
(b) 1 à 20 % en moles de motif de formule (II) suivante : 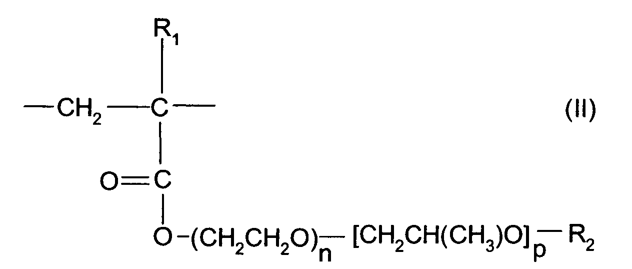 dans laquelle n et p indépendamment l'un de l'autre désignent un nombre entier allant de 0 à 24, sous réserve que n + p soit inférieur à 25 ; R₁ désigne un atome d'hydrogène, un radical alkyle linéaire ou ramifié comportant de 1 à 6 atomes de carbone, et R₂ désigne un radical alkyle linéaire ou ramifié, comportant de 6 à 30 atomes de carbone.

2. Composition selon la revendication 1, **caractérisée en ce que** le polymère est neutralisé partiellement ou totalement par une base minérale ou organique.

3. Composition selon la revendication 1 ou 2, **caractérisée en ce que** le polymère comprend de :
(a) 85 à 99 % en moles de motif acide 2-acrylamido 2-méthylpropane sulfonique (AMPS) de formule (I), et
(b) 1 à 15 % en moles de motif de formule (III) suivante : 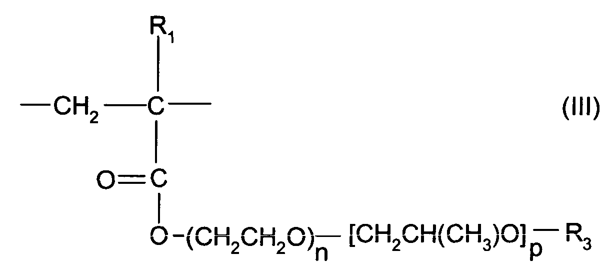
dans laquelle n et p indépendamment l'un de l'autre désignent un nombre entier allant de 7 à 24, sous réserve que n + p soit inférieur à 25 ; R₁ désigne un atome d'hydrogène, un radical alkyle linéaire ou ramifié comportant de 1 à 6 atomes de carbone et R₃ désigne un radical alkyle linéaire ou ramifié, comportant de 6 à 15 atomes de carbone.

4. Composition selon l'une quelconque des revendications 1 à 3, **caractérisée en ce que** le polymère est obtenu à partir d'acide 2-acrylamido-2-méthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène,
- d'un alcool oxo en C₁₁, oxyéthyléné par 8 moles d'oxyde d'éthylène,
- d'un alcool oxo en C₁₁ oxyéthyléné par 7 moles d'oxyde d'éthylène,
- d'un alcool en C₁₂-C₁₄ oxyéthyléné par 7 moles d'oxyde d'éthylène,
- d'un alcool en C₁₂-C₁₄ oxyéthyléné par 9 moles d'oxyde d'éthylène,
- d'un alcool en C₁₂-C₁₄ oxyéthyléné par 11 moles d'oxyde d'éthylène,
- d'un alcool en C₁₆-C₁₈ oxyéthyléné par 8 moles d'oxyde d'éthylène,
- d'un alcool en C₁₆-C₁₈ oxyéthyléné par 15 moles d'oxyde d'éthylène,
- d'un alcool en C₁₆-C₁₈ oxyéthyléné par 11 moles d'oxyde d'éthylène,
- d'un alcool en C₁₆-C₁₈ oxyéthyléné par 20 moles d'oxyde d'éthylène.

5. Composition selon la revendication précédente, **caractérisée en ce que** le polymère est choisi parmi le copolymère obtenu à partir de 91,5 % en moles d'AMPS et 8,5 % en moles de méthacrylate d'alcool en C₁₂-C₁₄ comportant 7 groupes oxyéthylénés ; le copolymère obtenu à partir de 92,65 % en moles d'AMPS et de 7,35 % en moles de méthacrylate d'alcool en C₁₆-C₁₈ comportant 8 groupes oxyéthylénés ; et leurs mélanges.

6. Composition selon l'une quelconque des revendications précédentes, **caractérisée en ce que** la quantité de polymère(s) amphiphile(s) va de 0,05 à 20 % en poids par rapport au poids total de la composition.

7. Composition selon l'une quelconque des revendications précédentes, **caractérisée en ce que** la phase huileuse représente de 15 % à 75 % en poids par rapport au poids total de la composition.

8. Composition selon l'une quelconque des revendications précédentes, **caractérisée en ce que** la quantité de cire(s) va de 0,1 à 10 % en poids par rapport au poids total de la composition.

9. Composition selon l'une quelconque des revendications précédentes, **caractérisée en ce que** la cire est choisie dans le groupe comprenant les cires minérales, les cires d'origine animale, les cires d'origine végétale, les huiles hydrogénées concrètes à 25°C, les esters gras et les glycérides concrets à 25°C, les cires synthétiques, les cires de silicone et leurs mélanges.

10. Composition selon l'une quelconque des revendications précédentes, **caractérisée en ce que** la cire est choisie parmi la cire de Carnauba, les cires de polyéthylène ayant une température de fusion commençante supérieure à 65°C, les cires microcristallines ayant une température de fusion commençante supérieure à 65°C, et leurs mélanges.

11. Composition selon l'une quelconque des revendications précédentes, **caractérisée par le fait qu'**elle est substantiellement exempte de tensioactif émulsionnant.

12. Composition selon l'une quelconque des revendications précédentes, **caractérisée par le fait qu'**elle constitue une composition cosmétique ou dermatologique.

13. Utilisation cosmétique d'une composition cosmétique selon l'une quelconque des revendications 1 à 11, pour le soin, la protection et/ou le maquillage de la peau et/ou des lèvres et/ou pour le soin des cheveux.

14. Procédé de traitement cosmétique de la peau, y compris du cuir chevelu, des cheveux, et/ou des lèvres, **caractérisé par le fait que** l'on applique sur la peau, les cheveux et/ou les lèvres, une composition cosmétique selon l'une quelconque des revendications 1 à 11.

15. Utilisation cosmétique d'une composition cosmétique selon l'une quelconque des revendications 1 à 11, pour le soin, la protection et/ou pour le maquillage des peaux sensibles.
